# EUROPEAN PATENT APPLICATION

(11) **EP 3 689 231 A1**
(43) Date of publication of application: **05.08.2020**
(21) Application number: 19154960.9
(22) Date of filing: 01.02.2019
(51) Int. Cl.: A61B 5/00, H04B 5/00, H01F 38/14, A61B 5/0215

(54) **POWER AND SIGNAL TRANSMISSION ON INTERVENTIONAL MEDICAL DEVICE**

(71) Applicant: Koninklijke Philips N.V., 5656 AG Eindhoven (NL)
(72) Inventor: KAHLERT, Joachim, 5656 AE Eindhoven (NL); VAN DER HORST, Arjen, 5656 AE Eindhoven (NL); VISSER, Cornelis Gerardus, 5656 AE Eindhoven (NL); HARBERTS, Dick, 5656 AE Eindhoven (NL); DOODEMAN, Gerardus Johannes Nicolaas, 5656 AE Eindhoven (NL); KLEIJNEN, Mark Peter Paul, 5656 AE Eindhoven (NL); MUELLER, Manfred, 5656 AE Eindhoven (NL)
(74) Representative: de Haan, Poul Erik

(57) **Abstract**

An interventional system is proposed enabling a contactless high frequency access and a high frequency data transfer on an existing medical device with a single conductive element like a wire or a metal core. Further, modules are provided which can be placed in between (or integrated in) available interventional devices.

## Description

### FIELD OF THE INVENTION

The invention generally relates to a medical system and a method for an interventional use of the same. Particularly, the invention relates to a guidewire and catheter system with a sensor at a distal portion thereof, adapted for high frequency powering and sensor read out.

### BACKGROUND OF THE INVENTION

Interventional guidewires are used, for example, for hemodynamic measurements (blood pressure, FFR). There are several burdens in the access to interventional devices like interventional guidewires and the transfer of data from the proximal access port to the distal location of a sensor and/or electronic circuitry.

The access to the interventional device, e.g. a diagnostic guidewire or sensor wire, is typically realized by a galvanic contact. The galvanic contact requires a plug and socket in the interface between the interventional device and the cathlab system. The galvanic contact has multiple drawbacks. Firstly, a socket, a wired connection is a burden in the clinical workflow, it hampers the manipulation (linear, rotational movement of the interventional device). Secondly, a galvanic connection is a hygiene issue in the clinical workflow.

The data transfer to the sensors and electronic circuitry at the distal location of the device raises multiple concerns. The attenuation and losses of electrical signals due to the tiny mechanical structures of e.g. a sensor wire/guidewire decrease the signal quality and the signal to noise ratio (SNR). Degradation of the electrical signals are through receiving and/or emitting electromagnetic radiation. The integration of electrical wires in the guidewire complicates the design and manufacturing and adds costs.

Single wire interfaces are already disclosed for interventional tools, like the inventions by "Guided Intervention". However, the size of a coupler/antenna is determined by the specified frequency band. Low frequencies in the range of kilo Hertz (as used e.g. in RFID tags or the wireless interface to a cardiac pace maker) need a huge antenna, which cannot be integrated in cardiovascular devices (catheter, guidewire).

### SUMMARY OF THE INVENTION

In view of the above-mentioned problems, it can be seen as an object of the invention to solve the above problems or at least to mitigate these problems.

These and further objects are solved by the subject-matter of the respective independent claims. Further embodiments are described in the respective dependent claims.

In general, an interventional system in accordance with the invention comprises a medical device with a single electrically conductive element, a high frequency coupler and a sensor. The electrically conductive element has a longitudinal axis and should be isolated. Further, an electrical shielding of the conductive element may be provided.

According to an aspect of the invention, the conductive element together with the isolation are movable along and around an axis of the medical device relative to the shielding. For example, the medical device may comprise a catheter with a lumen for accommodating a guidewire and a metal braiding providing an outer electrical shielding. A metal core of the guidewire may be the conductive element being movable within the lumen of the catheter, i.e. may be moved translationally and/or rotationally within the catheter.

The high frequency coupler may be adapted to generate a high frequency carrier, wherein the high frequency coupler may be arranged at a proximal portion of the medical device and may be coupled to the conductive element without mechanical contact, for example may be coupled without plug and socket.

The sensor may be provided with an application specific integrated circuit (ASIC) and an AC/DC conversion. The sensor and the AC/DC conversion may be adapted to be arranged at a distal portion of the medical device, wherein the AC/DC conversion may be adapted to receive the high frequency carrier of the high frequency coupler transmitted from the proximal portion over the conductive element to the distal portion, and may be adapted to provide DC energy for the application specific integrated circuit of the sensor. The application specific integrated circuit (ASIC) may control and may read out a sensing element of the sensor. Finally, a sensor signal may be transmitted back from the distal portion of the medical device over the conductive element to the proximal portion. The shielding provides a ground reference for the high frequency carrier and the transmitted signals.

The following aspects and advantages can be mentioned in connection with a system in accordance with the invention. An aspect of the invention is to enable a contactless high frequency access and a high frequency data transfer on an existing device with a single conductive element like a wire or a metal core. A further aspect provides modules, which can be placed inbetween (or integrated in) available interventional devices.

Beneficial features of devices as described herein are:
- Less mechanical, galvanic connectors are needed;
- Contactless power and signal coupling between sensor and cathlab system;
- Nearfield coupling with high coupling coefficient; robust against environmental noise;
- Narrowband coupler being robust against out of band noise;
- In-band power and data transfer;
- Bidirectional data transfer on single conductive element, no additional return path is needed;
- Movement tolerant coupler. Uninterrupted signal and power transfer during a linear and/or rotational navigation of the sensor;
- Single wire power and data transfer on electrical conductive and elongated element of a medical device like a guidewire or sensor wire;
- High frequency matched and terminated wire at distal end;
- No extra DC or AC return path is needed;
- High frequency carrier wave-guiding;
- Low radiation, low losses;
- Signal shielding against environmental signal/noise;
- Impedance matched high frequency carrier transmission by e.g. a core or single wire and metal braiding of catheter;
- Use to control the navigation and the repositioning of interventional devices, enabling:
   - Quantified metering of a linear movement of the sensor
   - Monitor & meter linear movement of distal sensor in blood vessel
   - Navigation and Reposition of sensor at earlier addressed positions
   - The linear movement can be correlated to a change in the high frequency pattern of the readout signal. This change of the frequency pattern does not interfere with the low frequency pattern of the sensed hemodynamic property.

A system for diagnosis and/or treatment of intravascular conditions in accordance with an embodiment may generally comprise an intravascular device with at least one electrical wire running from the proximal part to a distal sensor and/or actuator, wherein the at least one electrical wire through the intravascular device is connected to an external console forming one half of an electric loop, wherein e.g. at least one ECG electrode may be connected to the external console forming the other half of the electric loop with the loop being closed by currents running through the body of the patient.

According to an embodiment, the system may generally comprise a frequency splitter in a console that splits a low frequency ECG signal from a higher frequency sensor signal (whereby the frequency cut-off may be between 250 Hz and 5 kHz). An ECG-out port can be connected to a standard patient monitoring system. The system may also comprise a high frequency wired coupling from high frequency port of splitter to the signal generator or data transceiver of an interventional device. The system may provide a common grounding of the devices and a patient monitor, an integration and visualization of ECG signals and sensor data on one console, and ECG gated processing of sensor data. The couplers may provide a bidirectional coupling for a forward running high frequency carrier as power supply and for a backward running sensor signal modulated high frequency carrier.

In accordance with embodiments, a high frequency coupler may be attached to or integrated at a proximal end of the elongated conductive element. Alternatively or additionally, the coupler may be attached or integrated to a proximal end of a catheter or even at a proximal side of a hemostatic valve.

The medical device of a system in accordance with an embodiment may be a catheter or may at least comprise a catheter, wherein a shielding of the medical device may thus be a metal braiding of the catheter. A catheter as medical device may be any kind of interventional catheter for diagnostic or therapeutic purposes, for example for ablation, abrasion, extraction of tissue or blood clot from an inner cavity of a body, or for insertion of stents or other treatment elements. In connection with a catheter, the medical device with a sensing element at its distal end portion may be a guidewire or an interventional tool like a biopsy device, or any other device which is adapted to be introduced into a body cavity through a catheter lumen.

According to another embodiment, the medical device of the system may have a shielding formed by a metal coating on an isolation layer of the medical device. The coating may be arranged along the length of the medical device, at a proximal portion and/or at a distal portion of the medical device. The coating may also be arranged over the isolation of the elongated conductive element, i.e. a wire or core.

The medical device may be a guidewire and the elongated element may thus be an electrically conductive core of the guidewire. It will be understood that such a guidewire may additionally have a metal coating as an electrical shielding or may be used together with a catheter as mentioned above. In some applications, it may be advantageous to provide a guidewire with a metal coating and use the same together with a catheter having a metal braiding so that an inner conductive wire or core will be constantly electrically shielded even if the guidewire is moved relative to the catheter. As will be appreciated by a skilled person, a portion of the metal coating of the gudewire should be located in the lumen of the catheter. The braiding around the lumen of the catheter and the metal coating of the guidewire which is in the lumen of the catheter may thus form an electromagnetic coupler, coupling the ground reference of the catheter (metal braiding) to the ground reference of the guidewire (metal coating).

According to an embodiment, the application specific integrated circuit ASIC may further be adapted to digitize the sensor signal and to modulate the high frequency carrier by the digitized sensor signal. The modulated high frequency carrier may be an amplitude modulated high frequency carrier, a frequency modulated high frequency carrier or a phase modulated high frequency carrier.

According to a further embodiment, the sensor may include a micromachined transducer, e.g. capacitive or piezoelectric micromachined ultrasound transducers (CMUT, PMUT), or a conventional piezoelectric sensor. The sensors may be configured to provide at least one of a pressure measurement, flow velocity measurement, volumetric flow measurement, temperature, appearance of the anatomy by imaging. CMUT and PMUT are particularly suitable to provide multiple alternative or simultaneous types of measurements, as one and the same sensor element is suitable for example for ultrasound imaging of the anatomy such as vessel, and pressure measurement of blood in the vessel. Since flood flow velocity can be derived from ultrasound Doppler technique, and on the other hand volumetric flow can be derived from blood flow velocity and the geometry of the vessel from the ultrasound image, one and the same CMUT or PMUT sensor element is suitable for measurement of pressure, flow velocity, volumetric flow and appearance of the anatomy by ultrasound imaging.

It is to be noted that the sensor as described herein comprises a plurality of elements like a sensing element, a circuitry and a rectifier as power supply, and that these elements may be provided as partially or completely integrated assembly adapted to be arranged at a distal portion of a medical device. For example, the ASIC may be an integrated assembly together with a sensing element like a CMUT and may control the CMUT, may read out the CMUT and may digitize the CMUT signal, wherein an AC/DC converter or rectifier for energizing the ASIC may be a separate element. Depending on the intended application, it also may be of interest to integrate the powering, the control and the read out in a single assembly, but to provide the digitizing and the generation of a modulated high frequency carrier separately. Summarizing, the mentioning of parts of a sensor does not indicate whether the parts are provided separately or as an integrated assembly.

According to an embodiment, the sensor signal is transmitted along the medical device from the distal portion over the elongated element to the proximal portion by way of a modulated high frequency carrier.

According to an embodiment, the sensor signal is transmitted from the distal portion of the medical device at least partially through the body of a patient. It is also contemplated that the sensor signal is transmitted over the medical device as well as through the body of a patient.

It will be understood that the medical device may comprise a plurality of sensors. With a plurality of sensors, it may be possible to provide sensors sensing different aspects, e.g. pressure, temperature, electrical conductivity, flow velocity, volumetric flow. Different sensor may be implemented as utilizing different paths for a transmission of a sensor signal as well as utilizing different kinds of modulation of a frequency carrier for transmission.

According to an embodiment, the system further comprises a console for processing the sensor signal or sensor signals. The console may also be adapted to control the generation of the high frequency carrier. Alternatively or additionally, the console may be adapted to receive and process further signals, as for example an electrocardiogram signal from an ECG device which may be a part of the system in accordance with an embodiment. The electrocardiogram (ECG) device includes at least one ECG sensor, wherein the at least one ECG sensor may be adapted to receive not only electric impulses from the heart beat but also sensor signals transmitted from a sensor at a medical device transmitted through the body of the patient.

The high frequency coupler of the system may be at a catheter, at a hemostasis valve or at the medical device itself.

According to a further aspect, a method of use of any of the interventional systems as described above is provided, comprising the steps of generating a high frequency carrier, coupling the high frequency carrier to the elongated element of the medical device by means of the high frequency coupler, with the shielding of the elongated element as ground reference, transmitting the high frequency carrier over the elongated element, receiving the high frequency carrier by the AC/DC conversion of the sensor, converting the high frequency carrier so as to provide DC energy to the application specific integrated circuit of the sensor, controlling and reading out the sensing element of the sensor by means of the application specific integrated circuit, transmitting the sensor signal for further processing.

According to an embodiment, the method may further include the steps of digitizing the sensor signal, modulating the high frequency carrier by the digitized sensor signal, and transmitting the modulated high frequency carrier over the elongated element of the medical device. Alternatively or additionally, the method may comprise the step of transmitting the sensor signal through the body of the patient.

The aspects defined above and further aspects, features and advantages of the present invention may also be derived from the examples of embodiments to be described hereinafter and are explained with reference to examples of embodiments. The invention will be described in more detail hereinafter with reference to examples of embodiments but to which the invention is not limited. It is in particular noted that the above described embodiment are described based on specific and differing features and that a combination of those features, i.e. of these embodiments may also be of advantage.

### BRIEF DESCRIPTION OF THE DRAWINGS

Figure 1 illustrates aspects of a first embodiment of a system.
Figure 2 illustrates aspects of a second embodiment of a system.
Figure 3 illustrates aspects of a third embodiment of a system.
Figures 4 illustrates aspects of a fourth embodiment of a system.
Figure 5 illustrates aspects of a fifth embodiment of a system.
Figure 6 illustrates an electric concept of a sixth embodiment of a system.
Figure 7 illustrates an electric concept of a seventh embodiment of a system.
Figure 8 illustrates an electric concept of an eighth embodiment of a system.
Figure 9 illustrates modulation schemes of a signal.
Figure 10 illustrates aspects of a ninth embodiment of the system.
Figure 11 illustrates aspects of a tenth embodiment of the system.

The illustrations in the drawings are schematically only and not to scale. It is noted that similar elements are provided with the same reference signs in different figures, if appropriate.

### DETAILED DESCRIPTION OF EMBODIMENTS

The current invention concerns an interventional device, e.g. a diagnostic guidewire with a sensor that can be powered and read-out without any specific wiring. This results in a much better workflow and better hygiene. Because integration of electrical wires over the full length of the device is no longer needed, there is also potential for simplified design and manufacturing and lower costs.

Embodiments in accordance with the invention include the following elements: a medical device with a single electrically conductive wire or core that may act as a waveguide for powering a sensor at the distal portion and sending sensor signals back to the proximal end. A so-called RF coupler, i.e. a high frequency or radiofrequency coupler, that provides an electrical connection to the medical device. When provided as a capacitive coupler, the coupler may have the shape of a coaxial, rotation-symmetric tube. The sensor and related circuitry (e.g. in an ASIC) may be integrated in (the distal part of) the medical device. The circuitry may include the following functions: RF/DC conversion (better known as AC/DC conversion) to energize the sensor circuitry, DC voltage stabilization and an ASIC for controlling and reading the sensor. The sensor and circuitry may generate variations in reflected RF energy (e.g. through load modulation). This may lead to intermodulation signals that run over the medical device to the proximal side. These intermodulation signals may then be transferred to the evaluation electronics through the RF coupler.

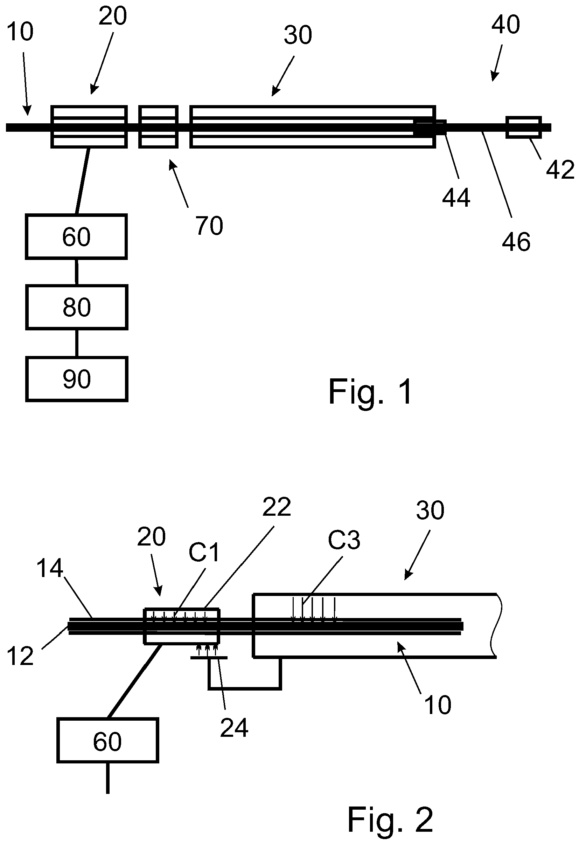

Fig. 1 illustrates the architecture of the described concept. The concept comprises a narrow band capacitive coupler 20 to access the core of a guidewire 10. The coupler 20 is driven by a circuit 60. An impedance matching circuit 70 may be provided to reduce reflection and insertion losses. The medical device 10 may be located within a lumen of a tubular member 30 so that a high frequency waveguiding may be provided by the tubular member 30 and the core of the medical device 10. At a distal portion of the medical device 10, a sensor 40 is provided with an AC/DC converter 44 to power up an ASIC sensor circuitry of e.g. a CMUT element 42 of the sensor 40. Sensor data from the sensor 40 may be digitised and a modulation of the carrier frequency may allow a signal readout through the coupler 20 at the proximal portion. A wired or a wireless interface 80 may connect the interventional medical device to a console 90.

Fig. 2 shows a capacitive coaxial coupler 20 and a proximal part of a tubular member like a catheter 30. The coupler 20 wirelessly couples power and signals to both the metal core 12 of the sensor wire 10 and to the tubular member 30, e.g. to a metal braiding which extends along the length of the tubular member and which surrounds an inner lumen thereof, in which lumen a medical device like a sensor wire or a guidewire may be movably arranged. Due to the symmetrical structure, the coupler 20 maybe denoted as coaxial coupler. Within the tubular member 30 and also within the coupler 20, the medical device 10 can be manipulated freely, at least linearly and/or rotationally.

Although the coupler can be widely moved, the electrical coupling remains uninterrupted. Some lines in Fig. 2 schematically illustrate the mentioned wireless coupling. Denoted with reference sign C1 is an electromagnetic field between the tubular element 22 of the high frequency or RF coupler 20 and the core 12 of the guidewire 10. Another electromagnetic field C3 is located between the catheter 30 and the core 12. The core 12 of the guidewire 10 may be surrounded by an isolation layer 14. In the embodiment of Fig. 2, an outer shielding of the tubular catheter 30 may also be connected to a separate capacitive coupling 24 providing a contactless connection of the coupler 20 to the catheter 30.

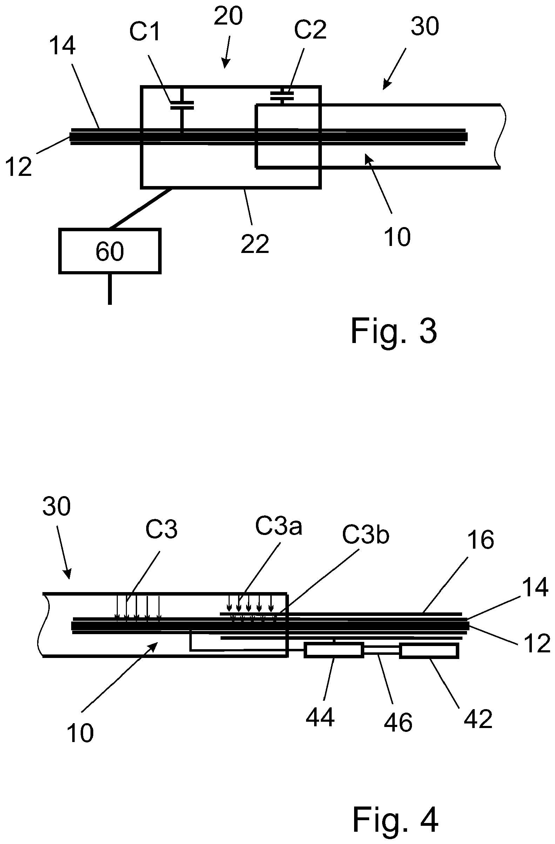

Fig. 3 shows another embodiment of a coupler 20 adapted to provide a radiofrequency or high frequency signal to both a guidewire 10 and a catheter 30. The coupler 20 of the embodiment of Fig. 3 includes a tubular element 22, which is sized to accommodate both the guidewire 10 and the catheter 30. Driven by the circuit 60, the tubular element 22 generates electromagnetic fields, one schematically drawn as a capacitor C1 between the tubular element 22 and the core 12 of the guide wire 10 and another one schematically drawn as a capacitor C2 between the tubular element 22 of the RF coupler 20 and a shielding of the catheter 30. It is noted that an electromagnetic high frequency field is understood to have capacitive as well as inductive aspects.

The electromagnetic coupling to the guidewire is formed by a cylindrical tube 22 around the guidewire 10. The length of the coupler 20 may typically be between a tenth and a quarter of the wavelength of the carrier frequency. For this cylindrical design, similar to a coaxial cable, the line impedance can be determined. The coaxial cable will be connected by impedance matching to the RF generator of the circuit 60. Such a RF coupler 20 can be a standalone device to be placed like a sleeve over the distal portion of the catheter and the guidewire, or may be integrated in either the guidewire 10, the catheter 30 or even a hemostatic valve (not shown).

In other words, the coupling to e.g. a metal braiding of the catheter 30 may be formed by a cylindrical tube 22 as shown in Figs. 2 and 3, or may be formed by a tube 22 around the guidewire 10 in combination with a galvanic connection to the metal braiding of the catheter as illustrated by the coupling 24 in Fig. 2.

The metal braiding of the catheter and the metal guidewire may be considered as forming a coaxial cable. If the line impedance of such a coaxial cable (catheter metal braiding - core wire of sensor wire) do not match the line impedance of coupler 20, an impedance matching circuit 70 can be added to provide a reflection-free interconnection between coupler 20 and catheter 30. The coaxial cable created by the catheter and the guidewire act as a wave guide to transfer a RF signal from the proximal coupler side to the distal side of the guide wire to the location of a sensor circuitry.

As shown in Fig. 4, a sensor circuitry including an application specific integrated circuit (ASIC) may be mounted at the distal portion of a guidewire 10. Fig. 4 illustrates the capacitive coupling between the ground reference of the ASIC and a metal shielding of a tubular member, here a catheter 30, which is used as the common ground reference. One of the input electrodes of the ASIC is connected to the core 12 of the guidewire. The second input electrode of the ASIC may be used as the ground reference for the ASIC and the sensor electronic. This ASIC ground reference may be wirelessly, i.e. capacitively and/or inductively coupled by electromagnetic field C3a to a metal braiding of the catheter 30 through an additional metal coating 16 on the guidewire 10. The metal coating 16 is coupled to the core 12 by way of an electromagnetic field C3b, in this embodiment. The metal core of the guidewire is shielded by an electrical isolation layer with a thickness of typically 10 - 50% of the radius of the metal core wire. At the distal end, the guidewire is coated with a metal layer over a length of 20 to 30 cm. The metal core and the metal layer form a coaxial cable. The ASIC ground reference is connected galvanically to the metal coating. During manipulation of the guidewire, a part of the distal end (the part which is coated by a metal layer) should remain within the catheter. At least a length of 3 - 5 cm of the metal coating should remain inside the catheter. This is because the overlapping part of the metal coating and the metal braiding of the catheter builds a coaxial capacitive coupler that couples the ground reference of the catheter to the ground reference of the ASIC/electronic circuitry.

In the proposed high frequency concept, the pressure may be measured with a CMUT sensor. When the pressure changes, the capacitance of the CMUT sensor changes. The CMUT sensor also contains an ASIC with an electrical circuit. This circuit converts the CMUT capacitance into e.g. a frequency modulated signal. As a result of this modulation, the current needed by the circuitry also modulates. This leads to a modulation in reflected high frequency energy, and this can be detected at the proximal end as intermodulation signals.

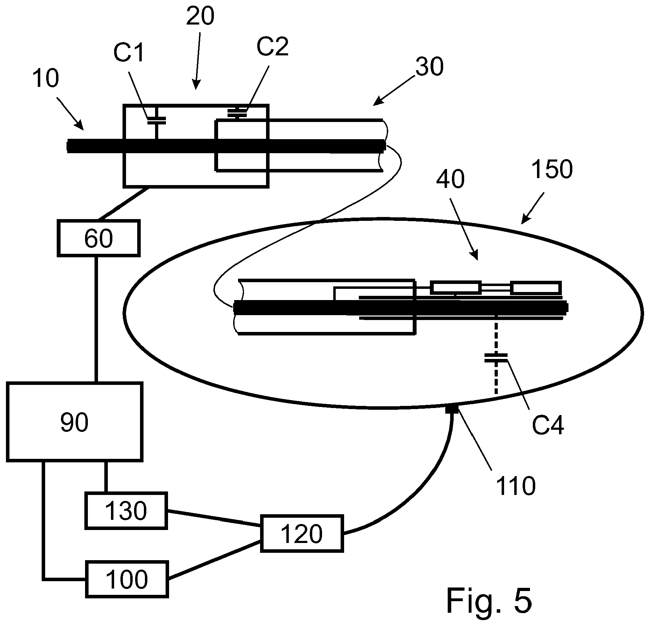

Fig. 5 shows an embodiment where the body 150 of the patient is used as common ground/return path. In this concept the metal coating 16 as described above is not needed. The common ground/return path if realized through capacitive coupling C4 between the body and an isolated part of the guidewire 10 near the tip. At the proximal end, also a coupling is to be realized.

A single unidirectional wire concept may be realized enabling unipolar communication with a sensor at the distal portion of an interventional device. The body may be used as the return path to close the electrical loop. To provide a return path with improved electrical properties, the return path may be split into two paths, a wireless body return path (C4) from the sensor to a patch 110 at the skin of the body and a wired path from the patch 110 back to the signal generator, which is connected to the proximal end of the interventional device.

The interconnection between signal generator and proximal end of the interventional device could be a wired or wireless communication link. A patch electrode 110 may be used to close the electrical return path for high frequency signals. The length of the body return path should be as short as possible and robust against patient movements and environmental disturbances. For a cardiac intervention, e.g in the coronary vessels, such a patch electrode shall be placed close to the heart at the anterior side of the patient's chest. For a peripheral vascular intervention, the patch may be placed in close vicinity to the peripheral vessel being treated. At least one ECG electrode may be used for the body return path. In cardiac interventions, ECG electrodes are fixed at the chest to measure the electrophysiological properties of the heart. These ECG signals are low frequency signals. The signals in the body return transmitted from the cardiac sensors are mid and high frequency signals. Thus, a multiplex frequency concept may be realized allowing the transport both of the low frequent ECG signal and the high frequency data signals via one ECG electrode 110. The signal may be transmitted by the ECG cable to an ECG device 130 which may be integrated in the console 90, e.g a patient monitor system. The signal received by the electrode 110 may be split by a demultiplexer 120 in an ECG signal transmitted to the ECG device 130 and in a signal from the interventional sensor transmitted to a sensor processing device 100. After demodulation, the ECG signals and the data from the interventional sensor can be processed separately by the existing systems.

After demodulation, the data of the sensor may be displayed and recorded at the console 90 of the interventional sensor system. Alternatively, an enhanced diagnostic and interventional device sensor data). An integration of both systems in one facilitates an easier ECG gating of the received sensor data. It will be understood that multiple ECG electrodes may enhance the robustness of the body return path and may eliminate signal distortion by a movement of the body.

With reference to Figs. 5 to 10, a concept is described where both powering and readout of a sensor at the distal end of an interventional device (guidewire) are done over a single conductive element like a wire or a guidewire core. The concept works as follows. The electrical power is high frequency (MHz-GHZ range) and is guided over the wire. This high frequency power is conditioned for the sensor circuitry through a converter (AC/DC or RF/LF). The signal from the sensor is converted into a binary pulse-length modulated signal (all at the distal end). This pulse-length modulated sensor signal is modulated on the high frequency carrier and transmitted backward on the single wire to the proximal coupler. These modulated high frequency carrier signals are readout through a wireless coupler at the proximal end and demodulated by a demodulator at the system console.

The following main elements are utilized to realize the concept according to an embodiment.

A coupler (without wire connection) is used to readout so-called Differential Binary Shift Keying (DBSK) modulated signals. The reason to use a differential binary shift keying modulation is twofold. First to get a DC free modulation by a differential shift keying and second using a binary shift keying to let toggle the modulated signal between two value. Such a toggling can be a binary change of an amplitude, a frequency or a phase of a carrier signal.

A demodulator for DBSK modulated signals can be implemented as a change detector determinig the point in time when the carrier signal changes in a discrete manner either its amplitude, or its frequency or its phase. A quantified analysis of change is not needed.

A wireless power supply unit, comprising AC/DC conversion or rectifier and voltage regulation may be used to power the sensor with circuitry, wherein a carrier frequency modulator may be integrated in the AC/DC converter circuitry.

Either a micromachined sensor (CMUT or PMUT) or a piezoelectric PZT sensor with digitized sensing and data quantification allows for sensing of physiological paramaters and/or ultrasound imgaging.

A binary data modulator for in-band return path or for body return path may be needed so as to be able to differentiate the sensor signal from the high frequence carrier signal on the one wire forming both signal transmission to the sensor and back.

According to an embodiment, the high frequency electrical power may be fed on a single wire (carrier). The high frequency may be converted to DC/lower frequency and conditioned to power the sensor. The sensor signal is digitized in the so-called sensor circuitry. The resulting digital signal is fed back to the single wire, resulting in a modulation in the same frequency band as the carrier. The sensor signal is extracted from the modulated carrier through a demodulator.

To achieve a noise robust system, a high frequency narrow-band communication of data and a transfer of power may be utilized. To improve the system against any drift, wandering and/or signal attenuation, a differential binary shift keying modulation (BSKM) maybe applied. The modulated signal toggles between two values. The point in time when the signal toggles is the information of relevance. Any signal change between the signal toggling is not relevant and is neither used nor analysed.

Elements and features of a disclosed embodiment are a high frequency wireless interface to interventional tools, a narrow-band wireless coupler at proximal interface, one single frequency for power supply and data transfer, an AC/DC power converter and voltage regulator embedded in resprespectively connected sensor ASIC, a digitization of the analogue sensor at the location of sensor, a carrier signal modulation at the sensor, embedded in a sensor ASIC, and an in-band modulation of carrier frequency. A digital modulation by differential binary shift keying may be at least one of a Differential Binary Amplitude Shift Keying (DBASK), a Differential Binary Frequency Shift Keying (DBFSK), a Differential Binary Phase Shift Keying (DBPSK). Finally, a sensor with electronic circuitry for digitization and an in-band readout of digital modulation may be provided.

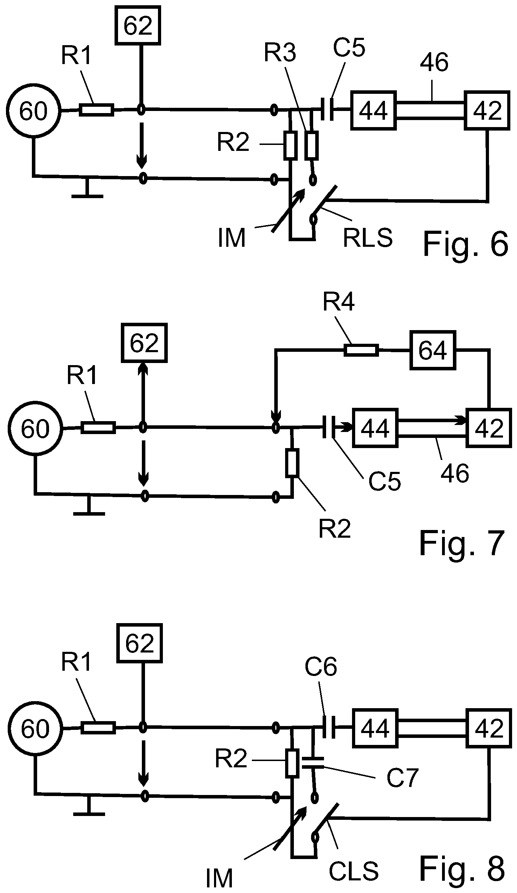

With reference to Figs. 6, 7 and 8, exemplary set-ups for the mentioned digital modulations are shown.

Fig. 6 Illustrates the design and embodiment of the DBASK, i.e. amplitude shift keying. At the proximal side (on the left) of the interventional device, the DBASK modulated signal is readout and demodulated by the demodulator 62. The demodulated signal may be recorded and shown at a console of the cathlab system (see e.g. Fig. 5). In this embodiment, the amplitude shift keying is realized by a resistive load switch RLS, which toggles the resistor of the line impedance termination with the input impedance modulation IM. The sensing element 42 in this embodiment is a CMUT element with an ASIC adapted to provide a pulse length encoded DC signal. R1 is a source resistance, R2 is a term resistance, and R3 is a load resistance. C5 illustrates a capacitive coupling.

Fig. 7 shows a design and embodiment realizing Differential Binary Frequency Shift Keying (DBFSK). The frequency shift keying may be realized by toggling the frequency of a voltage-controlled oscillator 64 with a related resistance R4.

Fig. 8 illustrates an embodiment by means of which a phase shift keying may be realized utilizing a capacitive load switch CLS. The change of the capacitive load changes the phase of the carrier signal.

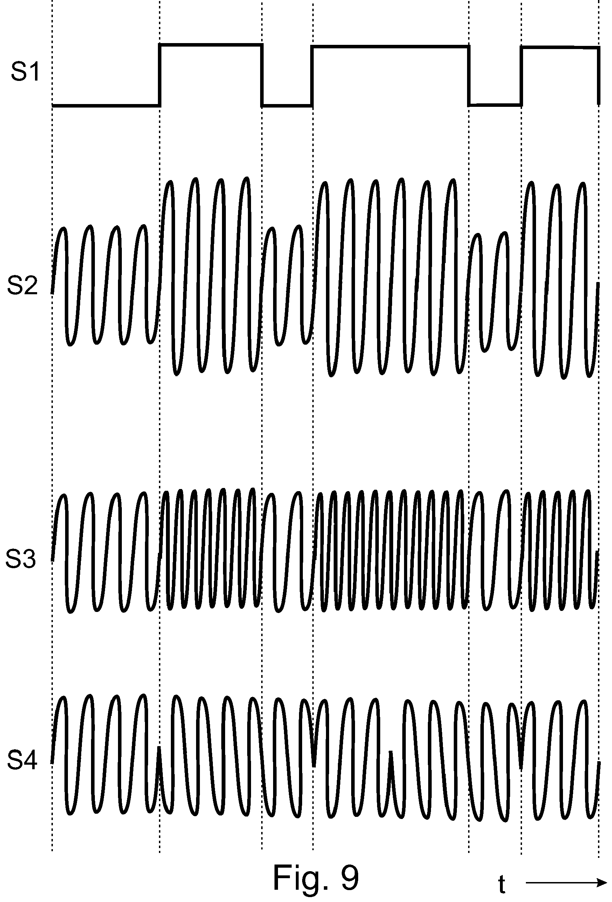

In all these embodiments, the sensor signal is digitized in the sensor circuit, the digitized value is coded by the length of a binary pulse in the time domain, and a pulse length modulated binary signal is used on the carrier signal. Fig. 9 illustrates an exemplary digitized sensor signal S1 as well as three different modulations thereof. The signal S2 shows a possible modulation of the amplitude as generated by the set-up of Fig. 6. The signal S3 shows a possible modulation of the frequency as generated by the set-up of Fig. 7. The signal S4 shows a possible modulation of the signal phase as generated by the set-up of figure 8.

In the following, examples of different sensor concepts are described for sensing the change of a physiological/hemodynamic parameter. These concepts illustrate how to quantify and digitize the sensed data.

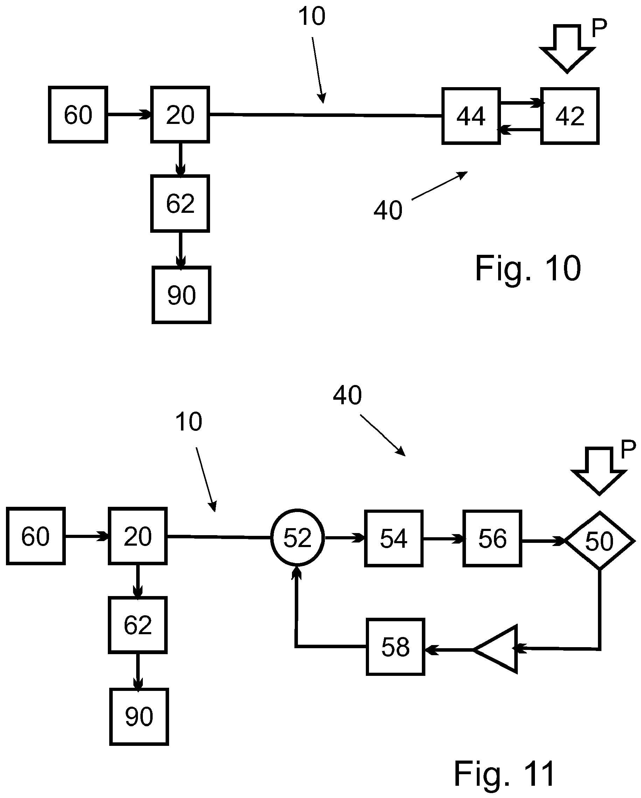

In Fig. 10, an exemplary embodiment for a CMUT ASIC sensor is disclosed. In this example, powering of the sensor 40 may be done with a frequency in the 400 MHz range. This high frequency is fed to the signal wire through a wireless coupler 60 and rectified at the distal end by the rectifier 44 for powering the sensor via connection 46. The digitization of the sensor signal by pulse length modulations may be done in the ASIC that is part of the CMUT sensor. In the example of Fig. 10, a frequency shift keying modulation technique (DBFSK) is used. This modulation signal is picked up with a wireless coupler 20, and converted for readout in a DC/low frequency signal by a demodulator 62.

As mentioned above, either an amplitude or a phase shift keying may be used, alternatively. According to an embodiment, a single wire may be used to transmit the modulated signal to the coupler 20 and the readout/modulation circuitry. A catheter that is surrounding the wire may act a electrical return path. Together with the wire it forms a coax transmission line. However, the electrical return signal may be radiated from the sensor ASIC and transmitted through the body (body return path) to a receiver integrated in the demodulator and readout circuit.

In Fig 11, a similar embodiment is described, now with a piezoelectric sensor. Powering of the sensor may again be done with a frequency in the 400 MHz range. This high frequency is fed to the signal wire through a wireless coupler 20, rectified by the rectifier 54 and regulated by voltage regulator 56 at the distal end for powering the piezoresistive sensor 50. The signal is digitized by converting the amplified sensor signal through a voltage controlled oscillator (VCO) 58 into a frequency modulated signal. This modulation signal is picked up again with a wireless coupler 20, and converted for readout in a DC/low frequency signal by a demodulator 62. A mixer 52 may be provided along the signal paths. A final processing of the sensor signal may be performed by console 90.

While the invention has been illustrated and described in detail in the drawings and foregoing description, such illustration and description are to be considered illustrative or exemplary and not restrictive; the invention is not limited to the disclosed embodiments. Other variations to the disclosed embodiments may be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

## Claims

1. An interventional system comprising:
a medical device (10) having a proximal portion and a distal portion, the medical device including an elongate element (12) extending along the medical device and at least partially in the proximal portion and the distal portion, wherein the elongate element is electrically conductive, an isolation layer (14) surrounding the elongate element, and an outer electrical shielding (16, 30), wherein the elongate element is movable relative to and within the shielding,
a high frequency coupler (20) for generating a high frequency carrier, wherein the high frequency coupler is arranged at a proximal portion of the medical device (10) and is electromagnetically coupled to the elongate element (12) of the medical device, and
a sensor (40) with a sensing element (42), an application specific integrated circuit and an AC/DC converter (44), wherein the sensor is arranged at a distal portion of the medical device,
wherein the AC/DC converter (44) of the sensor is adapted to receive the high frequency carrier of the high frequency coupler transmitted from the proximal portion over the elongate element to the distal portion, and to provide DC energy for the application specific integrated circuit of the sensor,
wherein the application specific integrated circuit is adapted to control the sensing element and to provide a sensor signal based on received measurement signals from the sensor element, and
wherein the shielding provides a ground reference for transmitted signals.

2. The system of claim 1, wherein the medical device (10) comprises a tubular member (30) including the shielding.

3. The system of claim 2, wherein the elongate element comprises an electrically conductive coating on the isolation layer, the coating being arranged at least at a distal portion of the elongate element.

4. The system of claim 3, wherein the elongate element is disposed within the tubular member, and the electrically conductive coating on the elongate element overlaps with the shielding in a longitudinal direction of the medical device at least for a length of 3 cm.

5. The system of any one of claims 1 to 4, wherein the application specific integrated circuit is further adapted to digitize the sensor signal and to modulate the high frequency carrier by the digitized sensor signal, wherein the modulated high frequency carrier includes at least one signal out of the group consisting of an amplitude modulated high frequency carrier, a frequency modulated high frequency carrier and a phase modulated high frequency carrier.

6. The system of any of the claims 1 to 5, wherein the sensing element (42) is configured to provide at least one of a physiological parameter measurement from the group of pressure, volumetric flow, flow velocity.

7. The system of claim 6, wherein the sensing element is configured to measure multiple physiological parameters.

8. The system of claim 6 or 7, wherein the sensing element is further configured to measure an appearance of the anatomy by imaging.

9. The system of any of the claims 6 to 8, wherein the sensing element is micromachined transducer from the group of capacitive and piezoelectric micromachined ultrasound transducer.

10. The system of any one of claims 1 to 9, wherein the sensor signal, when the system is in operation, is transmitted along the medical device (10) from the distal portion over the elongate element to the proximal portion.

11. The system of any one of claims 1 to 9, wherein the sensor signal, when the system is in operation, is transmitted from the distal portion of the medical device at least partially through the body (150) of a patient.

12. The system of any one of claims 1 and 11, further comprising a console (90) for processing the sensor signal.

13. The system of any of the claims 1 to 12, the medical device further comprising a hemostasis valve, wherein the high frequency coupler (20) is integrated in the hemostasis valve.

14. The system of any of claims 1 to 13, further comprising an electrocardiogram device (130) with at least one electrocardiogram sensor (110), wherein the at least one electrocardiogram sensor is adapted to receive the sensor signal transmitted from the sensor at the distal portion of the medical device through the body of the patient.

15. A method of use of an interventional system in accordance with any of the preceding claims, the method comprising the steps of:
generating a high frequency carrier,
coupling the high frequency carrier to the elongate element of the medical device (10) by means of the high frequency coupler (20), with the shielding of the elongate element as ground reference,
transmitting the high frequency carrier over the elongate element (12),
receiving the high frequency carrier by the AC/DC convertor of the sensor,
converting the high frequency carrier so as to provide DC energy to the application specific integrated circuit of the sensor (40),
controlling the sensing element of the sensor and providing the sensor signal based on received measurement signals from the sensor element by means of the application specific integrated circuit,
transmitting the sensor signal for further processing.
